# EUROPEAN PATENT APPLICATION

(11) **EP 3 586 618 A1**
(43) Date of publication of application: **01.01.2020**
(21) Application number: 18179402.5
(22) Date of filing: 22.06.2018
(51) Int. Cl.: A01K 29/00, A61B 5/00, A61B 5/11, A61B 5/16

(54) **DETERMINING A CONDITION OF AN ANIMAL**

(71) Applicant: United Pet Brands NV, 2000 Antwerpen (BE)
(72) Inventor: Ruparel, Apoorva, 400608 Thane West (IN)
(74) Representative: IP HILLS NV

(57) **Abstract**

Example embodiments describe a system (101) configured to determine a condition (200, 201) of an animal (100, 310), the system (101) comprising a motion sensor (503) configured to measure movements (110, 111) of a limb (103) when attached to the animal (100, 310); the system (101) further comprising an electronic library (504) comprising movement patterns related to respective conditions (200, 201); the system further comprising a controller (500) configured to obtain from the motion sensor (503) measured movements of the limb (103); to derive therefrom a movement pattern; and to select the related condition from the electronic library (504).

## Description

### Field of the Invention

The present invention generally relates to a system and method for determining a condition of an animal.

### Background of the Invention

Companion animals or pets are kept by persons for a variety of reasons, such as company, protection, and/or entertainment. Often, owners of a pet regard them as a part of their family or household and are given a dedicated position within a hierarchy of the household.

Moreover, owners attribute to their pet a personality, a character trait, and some degree of intelligence. The owner further interprets a behaviour of his pet by, for example, examining its posture, mobility, and/or facial expression. Through this interpretation, a condition or mood is attributed to the pet, such as, for example, happiness, anxiety or grief. The owner will then react to this mood by, for example, put at ease the pet or try to comfort it.

A problem, however, is that the owner trusts his own interpretation, although it may be wrong. When anticipating to a wrongly interpreted condition or mood, the animal may react in an unexpected manner, which may lead to dangerous situation, such as a punch or strike of the animal towards the owner. Another drawback is that the pet may not be aided at all, and that a bad condition, such as for example illness, is reinforced. Finally, an owner may determine a condition of an animal too late or not at all.

There is thus a need for a system and related method for determining a condition of an animal in a more efficient and accurate way.

### Summary of the Invention

It is therefore an object of the present disclosure to alleviate the above drawbacks and to provide an improved solution for determining a condition of an animal in a more efficient and accurate way.

This object is achieved, in a first aspect, by a system configured to determine a condition of an animal, the system comprising a motion sensor configured to measure movements of a limb when attached to the animal, the system further comprising an electronic library comprising movement patterns related to respective conditions, the system further comprising a controller configured to:
- obtain from the motion sensor measured movements of the limb;
- derive therefrom a movement pattern; and
- select the related condition from the electronic library.

The system comprises a motion sensor which is, for example, an accelerometer configured to measure movements in different directions. The motion sensor is attached to the animal and measures movements of a limb in the different directions. The movements are, for example, biomechanical motions of a limb and/or of the animal as a whole.

The system further comprises an electronic library which comprises movement patterns. A movement pattern is regarded as a way that a limb moves during a predefined time interval and comprises, for example, a position of the limb with respect to the body of the animal, a posture of the limb and/or how frequently and/or how fast the limb moves during the time interval. The movement patterns are thus related to the type of limb and each further related to a respective condition of an animal.

The system also comprises a controller which is configured to obtain from the motion sensor measured movements of the limb. The motion sensor may, for example, transfer the measurements to the controller in real-time, or may also comprise an internal memory unit such that measurements are stored during a time interval, and subsequently obtained by the controller during intervals.

The controller is further configured to derive from the obtained measured movements a movement pattern. In other words, the controller thus analyses and decrypts the obtained measurements, such that a movement pattern is derived therefrom.

Next, the controller relates the derived movement pattern with the movement patterns stored in the electronic library and selects therefrom the related condition. Differently formulated, by comparing the derived movement pattern with those stored in the library, the controller determines the condition of the animal. Finally, the condition may be reported to the owner.

Different advantages are identified. Firstly, the determination of the condition of the animal is based on objective measurements instead of an interpretation of the owner. Secondly, the condition of the animal may be determined even if the owner is not near the animal, for example, during working hours, and even during a whole day, thus also at night. Thirdly, a condition of the animal, or a change thereof, may immediately be reported to the owner, such that the owner may immediately take appropriate actions.

According to an embodiment, the condition comprises at least one of the group of a mood, a health parameter, an expression, and/or a temperament.

A health parameter comprises, for example, 'healthy', 'ill', 'tired', 'agile', or any other term indicating the health of the animal.

An expression may comprise words, for example, 'love', 'happy', 'peaceful', 'comfortable', 'panic', 'fear', or any other word that indicates the mood and/or condition of the animal.

An expression may also comprise phrases, for example, 'I'm hungry', 'I'm tired', 'I'm feeling comfortable', or any other phrase indicating the mood and/or condition of the animal.

A temperament comprises, for example, 'social', 'impulsive', 'melancholic', 'sad', or any other term that indicates the temperament of the animal.

The different terms of expression indicating the condition of the animal, thus a mood, a health parameter, a word, a phrase, and/or a temperament, may also be combined.

The condition may also be available in different languages.

Advantageously, by expressing the condition through a variety of terms of expression, the condition of the animal may be interpreted in an accurate manner, and to the owner it seems as the animal is speaking with him. The owner may thus in an interactive manner communicate with his animal.

According to an embodiment, the limb is a tail, an ear or a leg.

The motion sensor may thus measure the movements of a tail, of an ear or of a leg, or a combination thereof. The motion sensor may, for example, be attached to the back side of an animal and by measuring movements of the muscles on the backside, movements of, for example, the tail are derived therefrom. The motion sensor thus measures these movements in an indirect way. The motion sensor may also, for example, be attached behind an ear of the animal such that movements of the ear are indirectly measured. The motion sensor may also be attached directly to the limb from which the movements are measured.

Since a tail, an ear, or a leg of an animal are often very dynamic and mobile, a movement pattern may be derived from the movements of these active limbs in an accurate way, and thus the related condition may also be determined in an accurate way.

According to an embodiment, the electronic library is cloud-based.

In other words, the controller communicates with the electronic library over a network, whereby the electronic library is stored in the cloud. This way, the system operates in an energy efficient manner. Furthermore, since the electronic library is cloud-based, a large amount of movement patterns and related conditions may be stored in it. Another advantage is that data from different systems may be used and combined to enhance the content of the library by systematically updating and improving it. For example, new sets of movement patterns and related conditions may be input in the library, which may be performed at a remote distance with respect to the animal.

According to an embodiment, the motion sensor is attachable to the limb and/or incorporable in/on the animal.

The motion sensor may also be attached to the limb, for example, on an ear or a tail. Furthermore, it may also be incorporated in the animal, for example, under the skin of the animal, or by a piercing. This way, the motion sensor is attached in a secure and robust manner, without harming the animal.

According to an embodiment, the system further comprises
- a first apparatus comprising the motion sensor and a first wireless interface configured to send the measured movements; and
- a second apparatus comprising the controller and a second wireless interface configured to receive the measured movements.

The first apparatus thus comprises a wireless interface which sends the measured movements from the motion sensor to the wireless interface of the second apparatus. This way, the animal only has to wear the motion sensor and the wireless interface, and, in case, a memory unit and/or a battery to power the motion sensor and the wireless interface. The wireless interface may, for example, be a low energy Bluetooth interface, or any other protocol suitable for wirelessly transmitting signals.

The controller in the second apparatus receives the measurements from the first apparatus. The second apparatus may thus be located at a distance from the animal, and preferably in the range of the wireless interface of the first apparatus. The second apparatus may then further comprise the electronic library, or the controller may communicate with the library over a network when it is cloud-based.

It is an advantage that the animal only has to wear the motion sensor, thereby reducing a load of wearing the first apparatus, and that the deriving of the movement pattern is performed remotely with respect to the animal, such that the energy demand of measuring the movements of a limb is limited.

According to an embodiment, the second apparatus is a wireless portable communication device.

The second apparatus is thus, for example, a smart phone, a tablet, or another portable device that, for example, solely comprises the controller and wireless interface. Advantageously, the owner or any other person may get directly be reported of the condition of the animal, even if he is located at a distant location with respect to the animal.

According to an embodiment, the first apparatus comprises a belt or a ring configured to fit around the limb and/or the animal.

The first apparatus comprising the motion sensor may thus be incorporated in a ring, and the ring is configured to fit around, for example, a tail of the animal. Alternatively, the first apparatus may also be hooked or fastened to a belt, which is then fastened around a limb or around the animal. The attaching of the first apparatus to the animal may thus be adapted depending on the type of animal, and further on the size thereof, and thus also to the size of the limb.

According to an embodiment, the movements comprise positions and/or movement frequencies of the tail.

Thus, when the limb is a tail, the motion sensor measures a position and movement frequencies of the tail. Since movements of a tail of an animal, such as for example for a dog or a cat, indicates a condition in an accurate manner, advantageously, the condition of the animal is likewise derived in an accurate manner.

According to an embodiment, the motion sensor is further configured to track relocations of the animal.

In other words, the motion sensor may, for example, comprise a GPS tracking device to track movements of the animal. These tracking data may then further be combined with movements of a limb to determine the condition of the animal. The tracking data may further be reported to the owner, for example, on the second apparatus

According to an embodiment, the electronic library further comprises a set of animal breeds; and the movement patterns are further related to an animal bread; and the controller is further configured to select an animal breed, such that the condition is selected from the movement patterns related to the selected animal breed.

Differently formulated, per type of animal, such as, for example, a dog or a cat, a further classification is made per breed. Through the controller a breed is then selected thereby setting the proper set of movement patterns. This way, the condition is determined in an accurate manner taking into account the type of the animal, and by setting the breed, the size and/or typical characteristics of that breed as well.

According to a second aspect, the disclosure relates to a computer-implemented method for determining a condition of an animal, the method comprising the steps of:
- obtaining from a motion sensor movements of a limb of the animal;
- deriving therefrom a movement pattern;
- selecting from an electronic library comprising movement patterns related to respective conditions the related condition.

According to an embodiment, the computer-implemented method further comprises the steps of:
- tracking from the motion sensor relocations of the animal;
- monitoring the condition and/or relocation of the animal.

According to a third aspect, the disclosure relates to a computer program product comprising computer-executable instructions for performing the method according the second aspect when the program is run on a computer.

According to a fourth aspect, the disclosure relates to a computer readable storage medium comprising the computer product according to the third aspect.

### Brief Description of the Drawings

Some example embodiments will now be described with reference to the accompanying figures.
Fig. 1 illustrates an animal wearing a system for determining the condition of the animal;
Fig. 2 illustrates an animal having different conditions;
Fig. 3 illustrates a wireless portable communication device for monitoring a condition of an animal;
Fig. 4 illustrates a system comprising a first and a second apparatus for determining a condition of an animal;
Fig. 5 illustrates a system for determining a condition of an animal;
Fig. 6 illustrates steps performed for determining a condition of an animal; and
Fig. 7 illustrates a suitable computing system for performing steps according to example embodiments.

### Detailed Description of Embodiment(s)

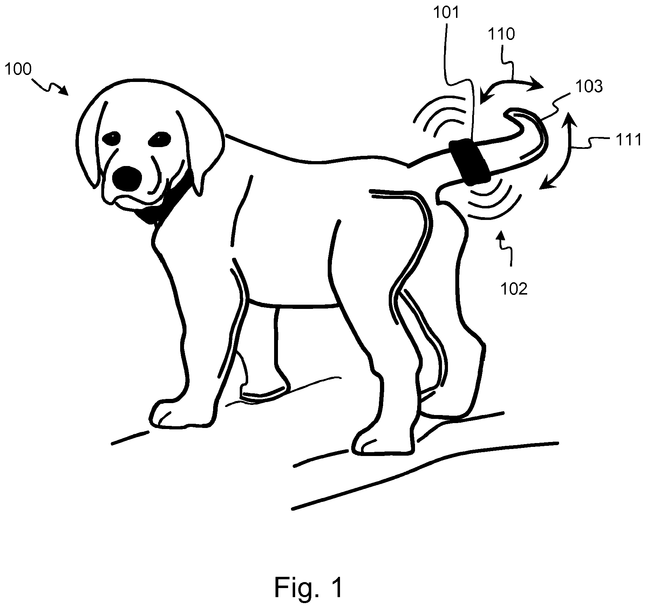

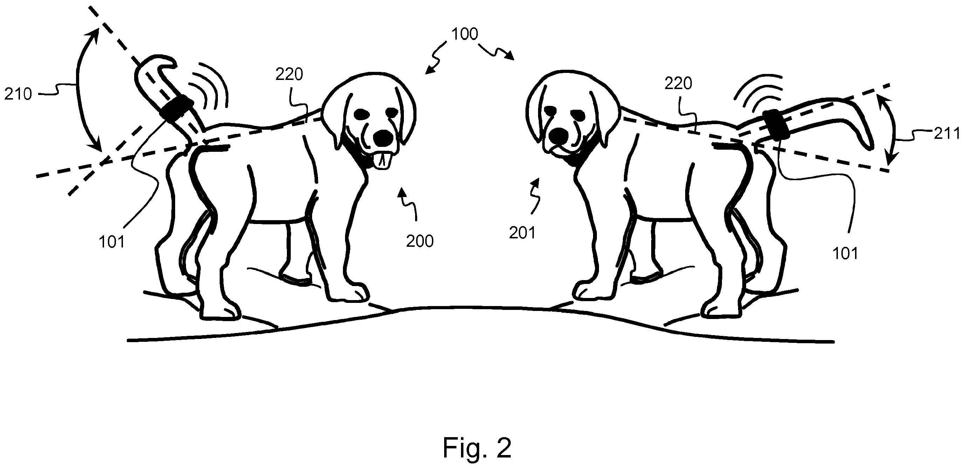

Fig. 1 illustrates an animal, and more in particular a dog 100, wearing a system 101 for determining a condition of the dog 100. The system 101 is attached to the tail 103 of the dog 100. The dog 100, as illustrated is Fig. 2, may have different conditions or moods, such as being happy 200 or being tranquil 201.

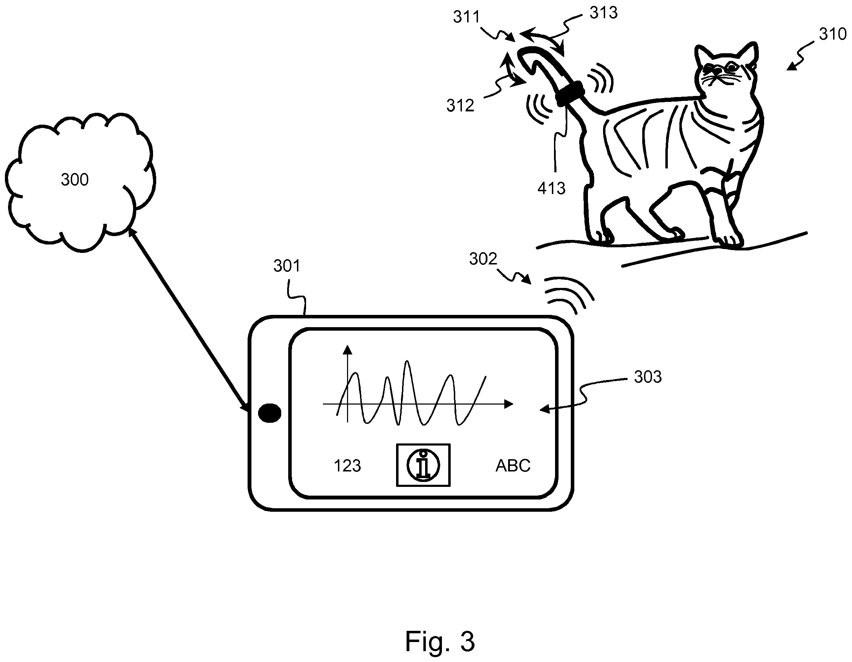

Besides a dog 100, the condition or mood of other animals may also be determined by the system 101, such as for example a cat 310, as illustrated in Fig. 3.

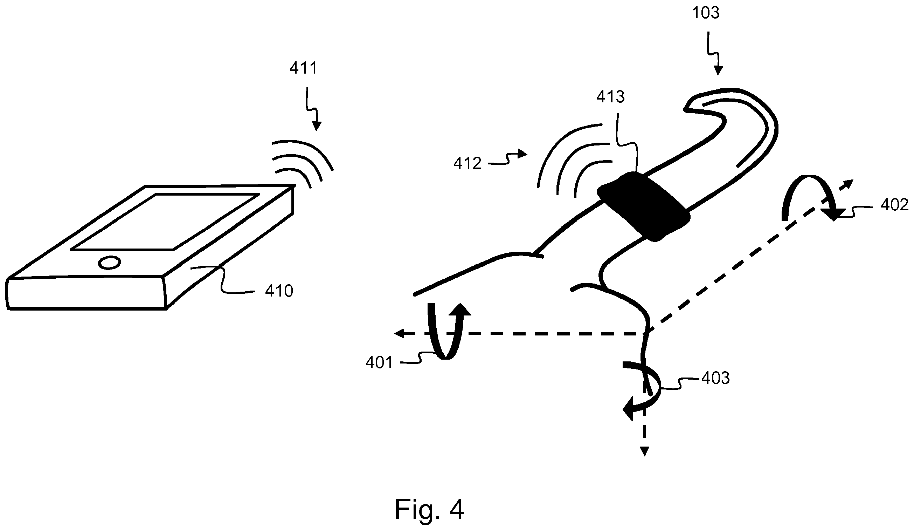

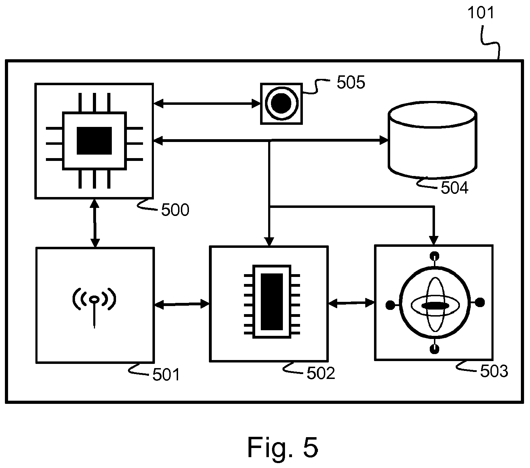

The system 101, as further illustrated in Fig. 5, comprises, according to an illustrative embodiment, a controller 500, a wireless interface 501, a memory unit 502, a motion sensor 503, a button 505, and an electronic library 504. According to another illustrative embodiment, the system 101 may comprise a first apparatus 413 and a second apparatus 410, as illustrated in Fig. 4. The first apparatus 413 comprises a motion sensor, similar to motion sensor 503, and a wireless interface, similar to wireless interface 501. The second apparatus 410 comprises a controller, similar to controller 500, and a wireless interface, similar to wireless interface 501.

The system 101, or in case the first apparatus 413, is attached to the dog 101 or the cat 310, and more in particular to the tail 103 or 311 thereof. In the illustrative embodiment of Fig. 1 the system 101 comprising the controller 500 and the motion sensor 503 is attached to the tail 103 of the dog 100, and in the illustrative embodiment of Fig. 2 the first apparatus 413 comprising a motion sensor and a wireless interface is attached to the tail 311 of the cat 310.

The motion sensor, such as motion sensor 503, is configured to measure movements of the tail 103 or 311 in different directions, such as directions 110 and 111 for the dog's tail 103, and directions 312 and 313 for the cat's tail 311. The motion sensor 503 comprises, for example, a three-axis accelerometer configured to measure movements in, for example as illustrated in Fig. 4, three directions of an orthogonal coordinate system 401, 402, and 403, or another coordinate system.

The measurements of the movements may, for example, be stored in the memory unit 502, or alternatively be send to the controller 500. In other words, the controller 500 obtains the measurements of the motion sensor 503 immediately, or through the memory unit 502.

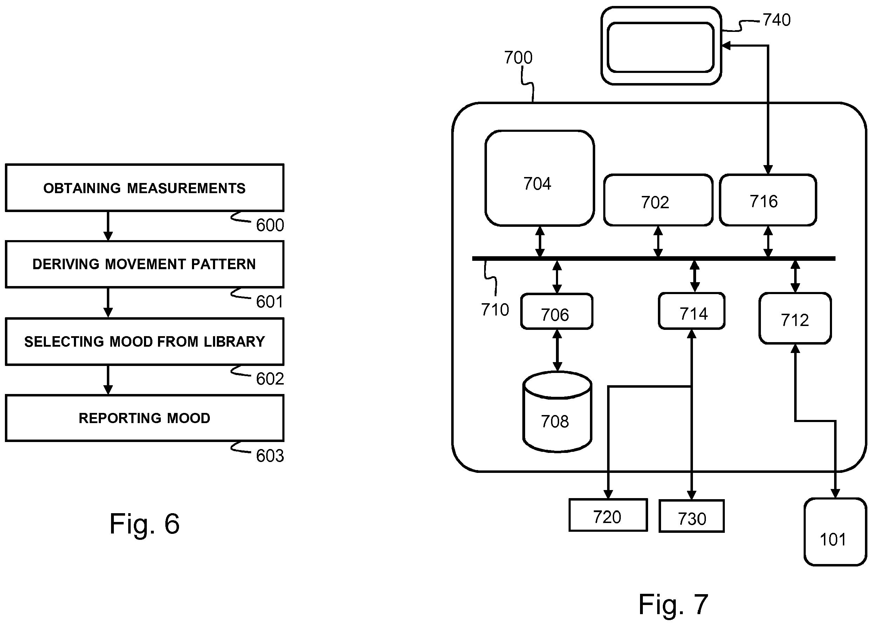

Thus, in a first step, as illustrated in Fig. 6, measurements are obtained 600 by the controller 500 from the motion sensor 503. Next, from these obtained 600 measurements, a movement pattern is derived 601, and more in particular for the illustrative embodiments of Fig. 1, 2, and 3, a tail's movement pattern is derived 601 by the controller 500. The system 101 may also be attached to another limb, for example, a leg or an ear, from which a movement pattern may also be derived 601.

A tail's movement pattern comprises, for example, the position or height of the tail, as illustrated by 210 and 211 for the dog 100, wherein the position 210 corresponds to a happy mood 200 of the dog 100, and wherein the position 211 corresponds to a tranquil mood 201 of the dog 100. The position is measured relative to the body of the animal, illustrated by reference 220. The movement pattern may further comprise how frequently and how fast the tail 103 moves during a predefined time interval.

Next, the movements of the tail 103 or 310 are interpreted, and more in particular the derived movement pattern therefrom. In other words, through the movements 110, 111, the dog 100 expresses a word, a condition, a language, and/or a mood. Likewise, for a cat 310, the movements 312 and 313 indicate also a word, a condition, a language, and/or a mood, which is interpreted by the system 101.

In the electronic library 504 of the system 101 a set of movement patterns is stored. The electronic library 504 may also be cloud-based 300, as illustrated in Fig. 3. Since the system 101 is configured to determine a condition of a variety of animals, such as dogs 100, or cats 310, a type of animal may be selected by, for example, a button 505 of the system 101, or through a user-interface 303 of an application running on a wireless device, such as a tablet 301, which remotely communicates with the system 101 via a wireless interface 302. Via the button 505, a user-interface 303, or any other system suitable for interacting with the system 101, an animal type is selected. Furthermore, within the electronic library 504, per type of animal, an animal breed may also be selected. For example, for a dog 100, a selection may, for example, be made between small dogs, such as, for example, Chihuahua to giant dog breeds like, for example, a Leonberger. This way, the characteristics of the animal are taken into account. For cats, a similar selection may be made, for example, from a regular domestic cat, to, for example, a Birman.

Subsequently, the derived 601 movement pattern is compared with the movement patterns stored in the electronic library 504. Through this comparison, from the electronic library 504 a mood is selected 602. Differently formulated, the movement patterns stored in the electronic library 504 are each related to a mood, and by selecting the movement pattern of the library 504 related to the derived 601 movement pattern, the related mood is selected 602.

The mood expresses the way the animal is feeling but may also express what the animal is trying to say. For example, the dog 100 may say, 'I'm hungry', by wagging his tail 103, which is interpreted by the system 101. This interpretation is then reported 603 in a final step to a person, for example the owner of the dog 100.

The reporting 603 may, for example, be performed by a wireless interface 501, such that the system 101 transmits 102 the selected word, mood, and/or condition of the electronic library 504 to a device configured for receiving the transmitted signal, such as a wireless portable communication device 410, which receives wirelessly 411 the transmitted 412 signals from the first apparatus 413.

In the illustrative embodiment of Fig. 3, the reporting 603 is performed on an application 303 of a tablet 301. A user may, for example, receive, besides the mood and/or condition of the animal 100, 310, a tracking path, and some other additional data, such as a name of the animal, an age, an historic diagram of the moods, and the data may by further be shared and/or exchanged on social media, such that an owner may share information of his pet with friends, family, and relatives.

Fig. 7 shows a suitable computing system 700 according to an example embodiment. Computing system 700 comprises means for performing the steps according to the above example embodiments. Computing system 700 may therefore be used as suitable implementation of system 101. Computing system 700 may in general be formed as a suitable general-purpose computer and comprise a bus 710, a processor 702, a local memory 704, one or more optional input interfaces 714, one or more optional output interfaces 716, a communication interface 712, storage element interface 706 and one or more storage elements 708. Bus 710 may comprise one or more conductors that permit communication among the components of the computing system 700. Processor 702 may include any type of conventional processor or microprocessor that interprets and executes programming instructions. Local memory 704 may include a random-access memory (RAM) or another type of dynamic storage device that stores information and instructions for execution by processor 702 and/or a read only memory (ROM) or another type of static storage device that stores static information and instructions for use by processor 702. Input interface 714 may comprise one or more conventional mechanisms that permit an operator to input information to the computing device 700, such as a keyboard 720, a mouse 730, a pen, voice recognition and/or biometric mechanisms, etc. Output interface 716 may comprise one or more conventional mechanisms that output information to the operator, such as a display 740, etc. Communication interface 712 may comprise any transceiver-like mechanism such as for example one or more Ethernet interfaces that enables computing system 700 to communicate with other devices and/or systems. The communication interface 712 of computing system 700 may be connected to such another computing system by means of a local area network (LAN) or a wide area network (WAN) such as for example the internet. Storage element interface 706 may comprise a storage interface such as for example a Serial Advanced Technology Attachment (SATA) interface or a Small Computer System Interface (SCSI) for connecting bus 710 to one or more storage elements 708, such as one or more local disks, for example SATA disk drives, and control the reading and writing of data to and/or from these storage elements 708. Although the storage elements 708 above is described as a local disk, in general any other suitable computer-readable media such as a removable magnetic disk, optical storage media such as a CD or DVD, -ROM disk, solid state drives, flash memory cards, ... may be used.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the scope of the claims are therefore intended to be embraced therein.

It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A system (101) configured to determine a condition (200, 201) of an animal (100, 310), the system (101) comprising a motion sensor (503) configured to measure movements (110, 111) of a limb (103) when attached to the animal (100, 310); the system (101) further comprising an electronic library (504) comprising movement patterns related to respective conditions (200, 201); the system further comprising a controller (500) configured to:
- obtain from the motion sensor (503) measured movements of the limb (103);
- derive therefrom a movement pattern; and
- select the related condition from the electronic library (504).

2. The system according to claim 1, wherein the condition comprises at least one of the group of a mood, a health parameter, an expression, and/or a temperament.

3. The system (101) according to any one of the preceding claims, wherein the limb (103) is a tail, an ear, or a leg.

4. The system (101) according to any of the preceding claims, wherein the electronic library (504) is cloud-based (300).

5. The system (101) according to any of the preceding claims, wherein the motion sensor (503) is attachable to the limb (103) and/or incorporable in/on the animal (100, 310).

6. The system (101) according to any one of the preceding claims, further comprising:
- a first apparatus (413) comprising the motion sensor (503) and a first wireless interface (412) configured to send the measured movements; and
- a second apparatus (410) comprising the controller (500) and a second wireless interface (411) configured to receive the measured movements.

7. The system (101) according to claim 6, wherein the second apparatus (410) is a wireless portable communication device (301).

8. The system (101) according to any of the claims 6 to 7, wherein the first apparatus (413) comprises a belt or ring configured to fit around the limb (103) and/or the animal (100, 310).

9. The system (101) according to any one of the claims 3 to 8, wherein the movements comprise positions and/or movement frequencies of the tail.

10. The system (101) according to any one of the preceding claims, wherein the motion sensor (503) is further configured to track relocations of the animal (100, 310).

11. The system (101) according to any one of the preceding claims, wherein the electronic library (504) further comprises a set of animal breeds; and wherein the movement patterns are further related to an animal breed; and wherein the controller (500) is further configured to select an animal breed, such that the condition is selected from the movements patterns related to the selected animal breed.

12. A computer-implemented method for determining a condition (200, 201) of an animal (100, 310), the method comprising the steps of:
- obtaining (600) from a motion sensor (503) movements of a limb (103) of the animal (100, 310);
- deriving (601) therefrom a movement pattern;
- selecting (602) from an electronic library (504) comprising movement patterns related to respective conditions (200, 201) the related condition.

13. The computer-implemented method according to claim 12, further comprising the steps of:
- tracking from the motion sensor (503) relocations of the animal (100, 310);
- monitoring the condition and/or relocation of the animal (100, 310).

14. A computer program product comprising computer-executable instructions for performing the method according to any one of the claims 12 to 13 when the program is run on a computer.

15. A computer readable storage medium comprising the computer program product according to claim 14
